(19) **Europäisches Patentamt** / **European Patent Office** / **Office européen des brevets**

(11) **EP 1 594 563 B1**

(12) **FASCICULE DE BREVET EUROPEEN**

(45) Date de publication et mention de la délivrance du brevet:
**14.05.2008 Bulletin 2008/20**

(51) Int Cl.:
***A61M 35/00*** *(2006.01)*

(21) Numéro de dépôt: **04712072.0**

(22) Date de dépôt: **18.02.2004**

(86) Numéro de dépôt international:
**PCT/FR2004/000365**

(87) Numéro de publication internationale:
**WO 2004/073783 (02.09.2004 Gazette 2004/36)**

(54) **APPLICATEUR DE PRODUIT LIQUIDE, PROCEDE ET MACHINE DE FABRICATION D'UN TEL APPLICATEUR**

APPLIKATOR FÜR FLÜSSIGPRODUKT UND VERFAHREN UND GERÄT ZUR HERSTELLUNG DIESES APPLIKATORS

LIQUID PRODUCT APPLICATOR AND METHOD AND MACHINE FOR PRODUCING ONE SUCH APPLICATOR

(84) Etats contractants désignés:
**AT BE BG CH CY CZ DE DK EE ES FI FR GB GR HU IE IT LI LU MC NL PT RO SE SI SK TR**

(30) Priorité: **19.02.2003 FR 0302039**

(43) Date de publication de la demande:
**16.11.2005 Bulletin 2005/46**

(73) Titulaire: **LEMOINE FRANCE**
**61430 Athis-de-l'Orne (FR)**

(72) Inventeur: **LEMOINE, Philippe**
**F-75016 Paris (FR)**

(74) Mandataire: **Casalonga, Axel**
**Bureau Casalonga & Josse**
**Bayerstrasse 71/73**
**80335 München (DE)**

(56) Documents cités:
**EP-A- 0 553 534       WO-A-89/10156**
**WO-A-02/083026       FR-A- 2 591 081**
**US-A- 3 661 666       US-A- 3 958 571**
**US-A- 4 740 194       US-A- 4 875 602**
**US-A- 5 702 035       US-B1- 6 343 717**

**EP 1 594 563 B1**

**Description**

L'invention concerne un applicateur de produit liquide. Elle se rapporte également à un procédé de fabrication d'un tel applicateur et à une machine de fabrication d'applicateurs correspondante.

Une application particulièrement intéressante de l'invention concerne la réalisation d'applicateurs se présentant sous la forme de cotons-tiges dont la tige est emplie d'un liquide et dont l'un des embouts d'ouate sert d'applicateur de produit.

Un tel applicateur comporte généralement une tige creuse formant réservoir de produit munie d'une extrémité ouverte destinée à son remplissage et d'une extrémité opposée fermée présentant une zone de moindre résistance mécanique, les dimensions de la tige étant telles que le produit soit maintenu à l'intérieur sous l'effet de la tension superficielle du produit, jusqu'à la cassure de la zone sécable. Pour procéder à la rupture de l'extrémité fermée, il convient de saisir manuellement l'embout correspondant et d'exercer un effort sur ce dernier tendant à casser la tige.

Généralement, les applicateurs de l'état de la technique de ce type sont pourvus de deux embouts d'extrémité identiques de sorte que l'embout correspondant à l'extrémité fermée est pourvu d'un indicateur identifiant le côté à casser. Cet indicateur est réalisé par exemple sous la forme d'un anneau de couleur formé sur l'embout concerné.

Le document US-A-5,702,035 divulgue un tel applicateur comportant une zone sécable. Le préambule de la revendication reprend les caractéristiques de l'applicateur décris dans ce document.

Ce type d'applicateur présente un certain nombre d'inconvénients majeurs. Tout d'abord, l'anneau de couleur n'est généralement pas situé de manière à indiquer précisément l'emplacement de la zone sécable, dans la mesure où il n'est prévu que pour indiquer le côté à casser. En effet, la zone sécable est généralement réalisée au cours d'une phase de matriçage au cours de laquelle on met en forme les embouts, par réalisation d'une zone de moindre résistance mécanique sur la tige elle-même, de sorte que la

ligne de moindre résistance mécanique ainsi formée n'est pas entourée par l'embout lui-même. Il en résulte un risque de blessure dans la mesure où, lors de la rupture, des portions tranchantes sont créées. C'est également la raison pour laquelle il apparaît des risques d'éclaboussure et de projection de produit lors de la rupture.

Le but de l'invention est de pallier ces inconvénients et de fournir un applicateur de produit liquide sous forme de bâtonnet, d'utilisation simplifiée et évitant tout risque de blessure et de projection de produit.

Selon l'invention, il est donc proposé un applicateur de produit liquide, du type comprenant une tige creuse qui est emplie dudit produit et qui est pourvue d'une première extrémité fermée, entourée d'un embout et présentant une zone sécable et d'une deuxième extrémité ouverte, la tige creuse étant adaptée pour que le produit soit maintenu à l'intérieur de la tige jusqu'à la cassure de l'extrémité fermée, l'embout comportant une zone de section rétrécie entourant la zone sécable.

En outre, cet applicateur comporte un deuxième embout prévu de manière à entourer la deuxième extrémité, cet embout constituant un embout applicateur du produit.

Dans différents modes de réalisation, le deuxième embout peut présenter diverses formes, telles que pointue, sphérique, aplatie, hélicoïdale, ou encore comporter une première partie sphérique prolongée axialement par une deuxième partie sensiblement tubulaire ayant une extrémité libre arrondie.

Pour la réalisation des embouts, on utilise, par exemple, du coton hydrophile, d'autres matériaux appropriés pour l'utilisation pouvant également être envisagés, tels que la viscose ou un polyester. En ce qui concerne le deuxième embout, la quantité de matière entrant dans sa constitution est déterminée en fonction de la quantité de liquide emplissant la tige.

De même, dans différents modes de réalisation, l'épaisseur de matière fibreuse au niveau de la section rétrécie du premier embout est comprise entre environ 0,5 et environ 3 mm. L'épaisseur de matière fibreuse au niveau de la section la plus grande du premier embout est comprise entre environ 1 mm et environ 20 mm.

La longueur de la tige peut être comprise entre environ 65 mm et environ 500 mm, de préférence comprise entre 70 mm et 75 mm.

En ce qui concerne le diamètre interne de la tige, celui-ci est compris entre environ 1 mm et environ 19 mm, de préférence compris entre 1 mm et 2,8 mm.

Le diamètre externe de la tige est, quant à lui, compris entre environ 2 mm et 20 mm, suivant le diamètre interne à obtenir.

Pour le bouchage de l'extrémité fermée, on utilise avantageusement une colle.

Enfin, on prévoit, de préférence, un bouchon de matière liquide au voisinage de l'extrémité ouverte de la tige, apte à éviter l'évaporation du produit liquide.

Selon l'invention, il est également proposé un procédé de fabrication d'un applicateur tel que défini ci-dessus, comprenant les étapes consistant à :

- transférer un ensemble de tiges creuses vers l'entrée d'un poste d'obturation ;
- obturer l'une des extrémités des tiges au moyen d'une colle ;

- emplir les tiges au moyen d'au moins un produit fluide, par leur extrémité opposée à l'extrémité obturée ; et
- fixer sur l'extrémité obturée de chaque tige un embout de matière fibreuse et, simultanément, former sur l'embout de matière fibreuse une zone de section rétrécie.

En outre, on réalise, dans la zone d'extrémité fermée, une zone sécable de moindre résistance mécanique dans la paroi de chaque tige, la zone de section rétrécie étant formée autour de la zone sécable.

Au cours de l'étape de fixation de l'embout, on fixe un embout applicateur de produit sur l'extrémité ouverte de chaque tige.

Dans un mode de mise en oeuvre, la fixation du ou des embouts comprend l'encollage d'au moins l'une des extrémités des tiges et l'enroulement d'une mèche de matière fibreuse sur la ou chaque extrémité enroulée.

Dans des applications particulières, le procédé selon l'invention peut comporter en outre une phase de conditionnement au cours de laquelle on emballe les applicateurs finis dans des emballages par ensembles d'au moins un applicateur, une phase de stérilisation des applicateurs finis pouvant en outre être prévue.

Enfin, selon l'invention, il est proposé une machine de fabrication d'applicateurs telle que définie ci-dessus, comprenant un poste d'obturation de tiges creuses pour l'obturation de l'une des extrémités mutuellement opposées des tiges, au moins un poste de remplissage des tiges obturées, un poste de formation d'embouts de matière fibreuse au moins sur l'extrémité fermée des tiges, et un convoyeur de transfert des tiges du poste d'obturation vers le poste de fixation, en passant par le poste de remplissage.

Le poste de formation d'embouts comporte un dispositif de formation d'une zone sécable de moindre résistance mécanique sur l'extrémité obturée des tiges et des moyens pour la formation d'une zone de section rétrécie sur l'embout, autour de la zone sécable.

D'autres buts, caractéristiques et avantages de l'invention apparaîtront à la lecture de la description suivante, donnée uniquement à titre d'exemple non limitatif, et faite en référence aux figures annexées sur lesquelles :

- la figure 1 est une vue en coupe d'un applicateur de produit liquide conforme à l'invention ;
- la figure 2 illustre un mode de réalisation particulier de l'embout applicateur ;
- la figure 3 illustre un autre mode de réalisation de l'embout applicateur ;
- la figure 4 illustre un quatrième mode de réalisation de l'embout applicateur ;
- la figure 5 illustre encore un autre exemple d'embout applicateur ;
- la figure 6 illustre encore un autre exemple d'embout applicateur
- la figure 7 est un schéma synoptique illustrant la constitution d'une machine de fabrication d'applicateurs selon l'invention ; et
- la figure 8 est un schéma illustrant la phase de remplissage des tiges.

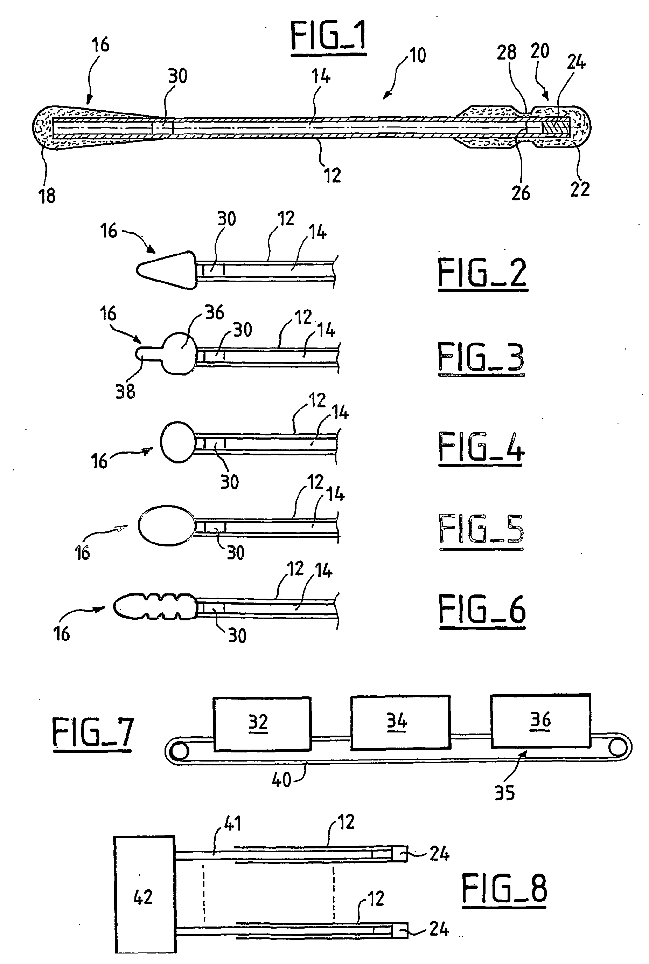

Sur la figure 1, on a représenté une vue en coupe longitudinale schématique d'un applicateur de produit liquide conforme à l'invention, désigné par la référence numérique générale 10. Sur cette figure, certains détails ont été exagérés par soucis de clarté.

Comme on le voit sur cette figure, l'applicateur 10 a une forme de coton-tige. Il comporte essentiellement un tube ou tige creuse 12 délimitant intérieurement un volume 14 empli de produit liquide et comportant une première extrémité 16 ouverte recouverte d'un embout applicateur 18 et d'une extrémité opposée 20 fermée recouverte d'un embout 22.

Le liquide contenu dans la tige peut être constitué par tout type de liquide correspondant à l'utilisation ou à l'application envisagée. On notera, à titre d'exemple, que le liquide contenu dans la tige peut être constitué par un produit cosmétique, un produit antiseptique, du liquide physiologique, ..., d'autres produits pouvant également être utilisés. En ce qui concerne le matériau entrant dans la constitution de la tige 12, celui-ci est par exemple réalisé en polypropylène, tout autre matériau compatible avec le liquide pouvant également être utilisé.

Les embouts 18 et 22 sont, quant à eux, par exemple réalisés en coton hydrophile. Ils peuvent également être réalisés en viscose et/ou en polyester, mais également en toute autre fibre cellulosique et/ou synthétique ou tout matériau absorbant, tel qu'une mousse, convenant pour l'application envisagée, des fibres différentes pouvant également être associées. De même, des associations fibres-matériau absorbant peuvent également être envisagées.

Comme on le voit sur la figure 1, l'extrémité 20 fermée est obturée par un bouchon 24 formé par dépôt d'une colle thermofusible. Toutefois, comme on le conçoit, cette obturation pourrait également être effectuée par fusion de l'extrémité à fermer. En raison des dimensions particulières de la tige 12, c'est-à-dire du faible diamètre interne de la tige, et de la tension superficielle du liquide, lors du stockage, le liquide emplissant la tige est maintenu à l'intérieur du volume 14, et ce, jusqu'à ce que l'extrémité 20 fermée de la tige soit mise à la pression atmosphérique. Pour ce faire, cette extrémité fermée 20 est pourvue d'une zone sécable 26 sous la forme d'une ligne de moindre résistance mécanique. Afin que cette zone sécable 26 puisse être repérée par un utilisateur, l'embout 22 de l'extrémité fermée 20 est pourvu d'une zone de section rétrécie 28 qui est formée lors de la réalisation de l'embout 20 par matriçage, de sorte que cette zone 28 se situe axialement au même niveau que la zone sécable 26 et entoure cette dernière.

Afin d'éviter une évaporation du liquide, un bouchon liquide 30 réalisé par exemple en huile, isole le liquide de l'atmosphère.

Comme on le voit sur les figures 2 à 4, l'embout applicateur 18 peut être réalisé de diverses formes, en fonction des applications.

Ainsi, par exemple, l'embout 18 peut avoir une forme pointue, par exemple conique pour une application très localisée, le diamètre de la surface délimitée par la pointe d'extrémité pouvant être de l'ordre de 1 mm.

De même, l'embout 18 peut être réalisé sous la forme d'une sphère (figure 3) ou encore, comme visible sur la figure 4, présenter une zone de section élargie afin d'éviter toute blessure en empêchant, par exemple, qu'il soit inséré profondément dans le conduit auditif d'un enfant en bas âge. Ainsi, à cet effet, l'embout 18 comporte une première portion 36 sphérique prolongée par une extrémité 38 généralement tubulaire ayant une extrémité libre arrondie. Enfin, comme on le voit sur les figures 5 et 6, respectivement, l'embout 18 peut présenter une forme aplatie dans une dimension pour faciliter l'application du produit sur une surface plus importante, ou présenter un ensemble de gorges annulaires disposées côte à côte. Bien entendu, d'autre formes peuvent également être envisagées.

On notera néanmoins que la quantité de matière fibreuse entrant dans la constitution de l'embout applicateur est liée à la quantité de liquide enfermé dans le volume 14. Par exemple, dans le cas où l'on utilise du coton, on pourra doter l'extrémité ouverte 16 d'une quantité de matière utile, c'est-à-dire une quantité de matière absorbant l'essentiel du liquide, de sorte que la quantité de liquide représente entre 5 et 20 fois le poids de coton. Cependant, une quantité de coton telle que le poids du liquide représente 15 fois le poids de coton permet de réaliser un applicateur permettant de restituer efficacement le liquide absorbé sans effet de mouillage trop important.

Par exemple, pour un applicateur ayant la forme d'un coton-tige de dimensions standard, la longueur de la tige est comprise entre 70 et 75 mm, son diamètre extérieur est compris entre 2 et 3 mm, et son diamètre intérieur est compris entre 1 et 2,8 mm.

Cependant, en fonction des applications, la longueur de la tige peut être comprise entre 65 et 500 mm, pour un diamètre extérieur compris entre 2 et 20 mm, et un diamètre intérieur compris entre 1 et 19 mm, en fonction du diamètre extérieur.

La tige peut, le cas échéant comporter une partie renflée délimitant un volume de réception de liquide. Elle peut également comporter une partie souple pour faciliter la distribution du liquide.

En ce qui concerne l'embout 22 entourant l'extrémité fermée 20, en fonction des applications, l'épaisseur de matière au niveau de la zone de section rétrécie est comprise entre 0,5 et 3 mm et, au niveau de la zone de section élargie, entre 1 et 20 mm, le diamètre de l'embout au niveau de la zone rétrécie étant bien entendu lié au diamètre de l'embout au niveau de la section agrandie.

A titre d'exemple spécifique, on réalise l'applicateur en utilisant une tige en polypropylène de longueur de 72,5 mm, ayant un diamètre extérieur de 2,6 mm, un diamètre intérieur de 1,9 mm et un embout ayant un diamètre extérieur de 4,7 mm et un diamètre au niveau du rétrécissement de 3,1 mm, l'embout applicateur ayant, quant à lui, l'une des formes illustrées aux figures 1 à 6.

Pour les raisons indiquées précédemment, la quantité de coton utilisé pour former l'embout applicateur est liée à la quantité de liquide emplissant la tige 12. Ainsi, par exemple, pour une longueur de liquide de 5,5 cm, la quantité Q de liquide enfermée dans la tige 12 est donnée par la relation suivante :

$$Q = \frac{\pi Di^2}{4} \cdot 5,5 \qquad (1)$$

dans laquelle Di désigne le diamètre intérieur de la tige

De manière conventionnelle, on utilise 0,03 g de coton pour la réalisation d'un embout. En considérant que la partie utile de l'embout correspond à un tiers de la masse de coton, que cette masse doit absorber 15 fois son poids pour obtenir un applicateur efficace, et que la masse volumique du liquide est celle de l'eau, le diamètre intérieur Di de la tige doit être donné par la relation suivante :

$$Di = \sqrt{\frac{4 \times 0,45}{3\Pi \times 5,5}} \qquad (2)$$

Le diamètre intérieur doit donc être de 0,186 cm.

L'applicateur qui vient d'être décrit, qui comporte une extrémité fermée munie d'une zone sécable entourée d'un embout pourvu d'une zone de section rétrécie entourant la zone sécable, et d'une extrémité opposée ouverte munie d'un embout applicateur dont la forme est adaptée à l'utilisation envisagée permet, d'une part, d'identifier aisément le côté à casser et de repérer la zone sécable et, d'autre part, d'éviter toute blessure et toute éclaboussure de produit lors de la rupture de l'extrémité fermée, la zone sécable étant entourée par l'embout. En outre, l'application est rendue aisée du fait de l'utilisation d'une quantité de matière fibreuse choisie en fonction de la quantité de liquide enfermé et donc du volume 14 interne de la tige.

En se référant aux figures 7 et 8, sur lesquelles on a représenté très schématiquement une installation de fabrication de tels applicateurs à grande cadence, on utilise une machine comprenant un poste 32 d'obturation de bâtonnet alimenté en tiges creuses ayant deux extrémités ouvertes, un ou plusieurs postes 34 de remplissage, en fonction du nombre de liquides différents à utiliser, un poste 35 de formation des embouts d'ouate, comprenant un poste 36 fixation de mèches d'ouate sur les extrémités mutuellement opposées de la tige, lequel poste de fixation comporte des moyens, connus en eux-mêmes, pour la mise en forme des embouts 18 et 22, ces postes étant reliés par un convoyeur 40.

Au niveau du poste d'obturation, l'une des extrémités des tiges est fermée par dépôt d'un bouchon de colle thermofusible ou, selon un autre mode de réalisation, par la fusion de l'extrémité correspondante de la tige. Après fermeture de l'une des extrémités des tiges, le convoyeur 40 transfère ces tiges vers le ou les postes de remplissage, au niveau duquel ils sont emplis d'un ou de plusieurs liquides puis du bouchon liquide. Dans le cadre de la présente description, et comme cela est régulièrement admis, par colle, on entend tout matériau convenant pour l'utilisation envisagée, notamment un matériau liquide lors de son application et passant à l'état solide, éventuellement sous l'action de conditions extérieures spécifiques, en acquérant des propriétés d'adhésion forte.

Comme on le voit sur la figure 8, ce remplissage s'effectue par lots de tiges, par exemple par lots de dix tiges, en utilisant des aiguilles, telles que 41, raccordées à une source 42 d'alimentation en liquide et animées de mouvements de va-et-vient. A l'issu du remplissage, on insère dans les tiges un bouchon liquide, par exemple un bouchon d'huile. Les tiges ainsi remplies sont transférées au poste de fixation d'ouate 36 au niveau duquel les embouts 18 et 22 sont formés. Ce poste de fixation d'ouate est pourvu d'un dispositif, de type classique en lui-même, permettant de réaliser la ligne 26 de moindre résistance mécanique. Au cours de cette phase, on réalise ainsi les zones de moindre résistance mécanique 26 sur les extrémités fermées des tiges. Dans un mode de réalisation avantageux, la réalisation des embouts s'effectue en faisant rouler des tiges sur une piste préalablement encollée par une colle thermofusible, puis en enroulant des mèches d'ouate sur les extrémités encollées. On pourrait également mettre en fusion les extrémités des tiges au lieu d'utiliser une colle. Simultanément, on effectue la mise en forme des embouts applicateurs et les zones de section réduite, qui sont formées autour des zones sécables. Cette phase est par exemple réalisée en utilisant des matrices appariées dont la conformation permet la réalisation des embouts, et en particulier la réalisation de la zone de section rétrécie dans l'embout 22.

En aval, des lignes de conditionnement, par exemple par emballage unitaire ou sous formes d'ensemble d'applicateurs et conditionnement des articles ainsi emballés dans des boîtes et, le cas échéant, un poste de stérilisation, peuvent être prévus.

On notera enfin que les postes d'obturation et de remplissage, d'une part, et les postes de fixation d'ouate et de matriçage, voire de conditionnement, d'autre part, peuvent être constituées par des unités de fabrication distinctes, implantées en des lieux géographiquement distants.

### Revendications

**1.** Applicateur de produit liquide, comprenant une tige creuse (12) qui est emplie dudit produit et qui est pourvue d'une première extrémité fermée (20), entourée d'un embout (22) et présentant une zone sécable (26) et d'une deuxième extrémité ouverte (16), la tige creuse étant adaptée pour que le produit soit maintenu à l'intérieur de la tige jusqu'à la cassure de l'extrémité fermée (20), **caractérisé en ce que** l'embout comporte une zone de section rétrécie (28) entourant la zone sécable.

**2.** Applicateur selon la revendication 1, **caractérisé en ce que** la deuxième extrémité est pourvue d'un deuxième embout (18).

**3.** Applicateur selon la revendication 2, **caractérisé en ce que** le deuxième embout (18) constitue un embout applicateur dudit produit.

**4.** Applicateur selon la revendication 3, **caractérisé en ce que** les premier et deuxième embouts sont réalisés en matière fibreuse.

**5.** Applicateur selon l'une quelconque des revendications 2 à 4, **caractérisé en ce que** le deuxième embout (18) est pointu.

**6.** Applicateur selon l'une quelconque des revendications 2 à 4, **caractérisé en ce que** le deuxième embout (18) est sphérique.

**7.** Applicateur selon l'une quelconque des revendications 2 à 4, **caractérisé en ce que** le deuxième embout (18) a une forme aplatie.

**8.** Applicateur selon l'une quelconque des revendications 2 à 4, **caractérisé en ce que** le deuxième embout comporte une première partie sphérique (36) prolongée axialement par une deuxième partie (38) sensiblement tubulaire ayant une extrémité libre arrondie.

**9.** Applicateur selon l'une quelconque des revendications 1 à 8, **caractérisé en ce que** la quantité de matière entrant dans la constitution du deuxième embout (18) est déterminée en fonction de la quantité de liquide emplissant la tige.

**10.** Applicateur selon l'une quelconque des revendications 1 à 9, **caractérisé en ce que** les embouts (18, 22) sont réalisés à partir d'une matière choisie parmi le coton hydrophile, la viscose, et un polyester.

**11.** Applicateur selon l'une quelconque des revendications 4 à 10, **caractérisé en ce que** l'épaisseur de matière fibreuse au niveau de la section rétrécie (28) du premier embout est comprise entre environ 0,5 et environ 3mm.

**12.** Applicateur selon l'une quelconque des revendications 4 à 11, **caractérisé en ce que** l'épaisseur de matière fibreuse au niveau de la section la plus grande du premier embout (28) est comprise entre environ 1 mm et environ 20mm.

**13.** Applicateur selon l'une quelconque des revendications 1 à 12, **caractérisé en ce que** la longueur de la tige (12) est comprise entre environ 65mm et environ 500mm, de préférence comprise entre 70 mm et 75mm.

**14.** Applicateur selon l'une quelconque des revendications 1 à 13, **caractérisée en ce que** le diamètre interne de la tige (12) est compris entre environ 1mm et environ 19mm, de préférence compris entre 1 mm et 2,8mm.

**15.** Applicateur selon l'une quelconque des revendication 1 à 13, **caractérisé en ce que** le diamètre externe de la tige (12) est compris entre environ 2 mm et 20mm.

**16.** Applicateur selon l'une quelconque des revendications 1 à 15, **caractérisé en ce que** ladite première extrémité de la tige (20) est fermée au moyen d'une colle.

**17.** Applicateur selon l'une quelconque des revendications 1 à 16, **caractérisé en ce qu'**il comporte en outre un bouchon (30) de matière liquide au voisinage de l'extrémité ouverte de la tige, apte à éviter l'évaporation du produit liquide.

**18.** Procédé de fabrication d'un applicateur selon l'une quelconque des revendications 1 à 17, **caractérisé en ce qu'**il comporte les étapes consistant à :

- transférer un ensemble de tiges creuses vers l'entrée d'un poste d'obturation (32) ;
- obturer l'une des extrémités des tiges au moyen d'une colle ;
- emplir les tiges au moyen d'au moins un produit fluide, par leur extrémité opposée à l'extrémité obturée ;
- fixer sur l'extrémité obturée de chaque tige un embout (22) de matière fibreuse et, simultanément, former sur l'embout de matière fibreuse une zone (28) de section rétrécie,

et **en ce qu'**il comporte en outre une étape de réalisation, dans la zone d'extrémité fermée, d'une zone sécable (26) de moindre résistance mécanique dans la paroi de chaque tige, la zone de section rétrécie étant formée autour de la zone sécable.

**19.** Procédé selon la revendication 18, **caractérisé en ce qu'**au cours de l'étape de fixation de l'embout, on fixe un embout (18) applicateur de produit sur l'extrémité ouverte de chaque tige.

**20.** Procédé selon l'une des revendications 18 et 19, **caractérisé en ce que** fixation du ou des embouts comprend l'encollage d'au moins l'une des extrémités des tiges et l'enroulement d'une mèche de matière fibreuse sur la ou chaque extrémité encollée.

21. Procédé selon l'une quelconque des revendication 18 à 20, **caractérisé en ce qu'**il comporte en outre une phase de conditionnement au cours de laquelle on emballe les applicateurs finis (10) dans des emballages par ensembles d'au moins un applicateur.

22. Procédé selon l'une quelconque des revendications 18 à 21, **caractérisé en ce qu'**il comporte en outre une phase de stérilisation des applicateurs finis.

23. Machine de fabrication d'applicateurs selon l'une quelconque des revendications 1 à 17, comportant un poste (32) d'obturation de tiges creuses pour l'obturation de l'une des extrémités mutuellement opposées des tiges, au moins un poste (34) de remplissage des tiges obturées, un poste (35) de formation d'embouts de matière fibreuse au moins sur l'extrémité fermée des tiges, et un convoyeur (40) de transfert des tiges du poste d'obturation vers le poste de fixation, en passant par le poste de remplissage, **caractérisée en ce que** le poste de formation d'embouts (35) comporte un dispositif de formation d'une zone sécable (26) de moindre résistance mécanique sur l'extrémité obturée des tiges et des moyens pour la formation d'une zone de section rétrécie sur l'embout, autour de la zone sécable.

**Claims**

1. Liquid product applicator, comprising a hollow wand (12) which is filled with said product and which is equipped with a first sealed end (20), surrounded by a tip (22) and having a breakable zone (26) and a second open end (16), the hollow wand being adapted so that the product is kept inside the wand until breaking of the sealed end (20), **characterized in that** the tip comprises a zone of narrowed cross section (28) surrounding the breakable zone.

2. Applicator according to Claim 1, **characterized in that** the second end is equipped with a second tip (18).

3. Applicator according to Claim 2, **characterized in that** the second tip (18) constitutes an applicator tip for said product.

4. Applicator according to Claim 3, **characterized in that** the first and second tips are produced from a fibrous material.

5. Applicator according to any one of Claims 2 to 4, **characterized in that** the second tip (18) is pointed.

6. Applicator according to any one of Claims 2 to 4, **characterized in that** the second tip (18) is spherical.

7. Applicator according to any one of Claims 2 to 4, **characterized in that** the second tip (18) has a flattened shape.

8. Applicator according to any one of Claims 2 to 4, **characterized in that** the second tip comprises a first spherical part (36) extended axially by a second substantially tubular part (38) having a rounded free end.

9. Applicator according to any one of Claims 1 to 8, **characterized in that** the amount of material incorporated into the composition of the second tip (18) is determined as a function of the amount of liquid filling the wand.

10. Applicator according to any one of Claims 1 to 9, **characterized in that** the tips (18, 22) are made from a material chosen from absorbent cotton, viscose and a polyester.

11. Applicator according to any one of Claims 4 to 10, **characterized in that** the thickness of the fibrous material at the narrowed cross section (28) of the first tip is between around 0.5 mm and around 3 mm.

12. Applicator according to any one of Claims 4 to 11, **characterized in that** the thickness of fibrous material at the largest cross section of the first tip (22) is between around 1 mm and around 20 mm.

13. Applicator according to any one of Claims 1 to 12, **characterized in that** the length of the wand (12) is between around 65 mm and around 500 mm, preferably between 70 mm and 75 mm.

14. Applicator according to any one of Claims 1 to 13, **characterized in that** the inner diameter of the wand (12) is between around 1 mm and around 19 mm, preferably between 1 mm and 2.8 mm.

**15.** Applicator according to any one of Claims 1 to 13, **characterized in that** the outer diameter of the wand (12) is between around 2 mm and 20 mm.

**16.** Applicator according to any one of Claims 1 to 15, **characterized in that** said first end (20) of the wand is sealed by means of an adhesive.

**17.** Applicator according to any one of Claims 1 to 16, **characterized in that** it additionally comprises a plug (30) of liquid material in the vicinity of the open end of the wand, capable of preventing the evaporation of the liquid product.

**18.** Process for manufacturing an applicator according to any one of Claims 1 to 17, **characterized in that** it comprises the steps consisting in:

- transferring a set of hollow wands towards the entrance of a sealing station (32);
- sealing one of the ends of the wands by means of an adhesive;
- filling the wands by means of at least one fluid product, via their end opposite to the sealed end; and
- attaching to the sealed end of each wand a tip (22) of fibrous material and, simultaneously, forming on the tip of fibrous material a zone (28) of narrowed cross section,

and **in that** it additionally comprises a step of producing, in the sealed end zone, a breakable zone (26) of lower mechanical strength in the wall of each wand, the zone of narrowed cross section being formed around the breakable zone.

**19.** Process according to Claim 18, **characterized in that** during the step of attaching the tip, a product applicator tip (18) is attached to the open end of each wand.

**20.** Process according to either of Claims 18 and 19, **characterized in that** attachment of the tip or tips comprises the application of adhesive to at least one of the ends of the wands and wrapping a tuft of fibrous material over the or each adhesive-coated end.

**21.** Process according to any one of Claims 18 to 20, **characterized in that** it additionally comprises a packaging phase during which the finished applicators (10) are packaged in packages in sets of at least one applicator.

**22.** Process according to any one of Claims 18 to 21, **characterized in that** it additionally comprises a phase of sterilizing the finished applicators.

**23.** Machine for manufacturing applicators according to any one of Claims 1 to 17, comprising a hollow wand sealing station (32) for sealing one of the mutually opposite ends of the wands, at least one station (34) for filling the sealed wands, a station (35) for forming tips of fibrous material at least on the sealed end of the wands, and a conveyor (40) for transferring the wands from the sealing station to the attachment station, passing through the filling station, **characterized in that** the station (35) for forming the tips comprises a device for forming a breakable zone (26) of lower mechanical strength on the sealed end of the wands and means for forming a zone of narrowed cross section on the tip, around the breakable zone.

**Patentansprüche**

**1.** Applikator für Flüssigprodukt, der eine hohle Stange (12) enthält, die mit dem Produkt gefüllt ist und mit einem ersten verschlossenen Ende (20), das von einem Endstück (22) umgeben ist und einen zerbrechbaren Bereich (26) aufweist, und einem zweiten offenen Ende (16) versehen ist, wobei die hohle Stange so ausgeführt ist, dass das Produkt bis zum Zerbrechen des verschlossenen Endes (20) im Inneren der Stange gehalten wird, **dadurch gekennzeichnet, dass** das Endstück einen Bereich mit verengtem Querschnitt (28) aufweist, der den zerbrechbaren Bereich umgibt.

**2.** Applikator nach Anspruch 1, **dadurch gekennzeichnet, dass** das zweite Ende mit einem zweiten Endstück (18) versehen ist.

**3.** Applikator nach Anspruch 2, **dadurch gekennzeichnet, dass** das zweite Endstück (18) ein Endstück zum Aufbringen des Produkts bildet.

**4.** Applikator nach Anspruch 3, **dadurch gekennzeichnet, dass** das erste und das zweite Endstück aus einem Fasermaterial hergestellt sind.

5.  Applikator nach einem der Ansprüche 2 bis 4, **dadurch gekennzeichnet, dass** das zweite Endstück (18) spitz zulaufend ist.

6.  Applikator nach einem der Ansprüche 2 bis 4, **dadurch gekennzeichnet, dass** das zweite Endstück (18) kugelförmig ist.

7.  Applikator nach einem der Ansprüche 2 bis 4, **dadurch gekennzeichnet, dass** das zweite Endstück (18) eine abgeflachte Form aufweist.

8.  Applikator nach einem der Ansprüche 2 bis 4, **dadurch gekennzeichnet, dass** das zweite Endstück einen ersten kugelförmigen Teil (36) aufweist, der axial durch einen im Wesentlichen röhrenförmigen zweiten Teil (38) mit einem freien abgerundeten Ende verlängert wird.

9.  Applikator nach einem der Ansprüche 1 bis 8, **dadurch gekennzeichnet, dass** die im Aufbau des zweiten Endstücks (18) enthaltende Materialmenge in Abhängigkeit von der die Stange füllenden Flüssigkeitsmenge bestimmt wird.

10. Applikator nach einem der Ansprüche 1 bis 9, **dadurch gekennzeichnet, dass** die Endstücke (18, 22) aus einem Material hergestellt sind, das unter Watte, Viskose und einem Polyester ausgewählt ist.

11. Applikator nach einem der Ansprüche 4 bis 10, **dadurch gekennzeichnet, dass** die Dicke des Fasermaterials an dem verengten Querschnitt (28) des ersten Endstücks zwischen ca. 0,5 und ca. 3 mm liegt.

12. Applikator nach einem der Ansprüche 4 bis 11, **dadurch gekennzeichnet, dass** die Dicke des Fasermaterials am größten Querschnitt des ersten Endstücks (28) zwischen ca. 1 mm und ca. 20 mm liegt.

13. Applikator nach einem der Ansprüche 1 bis 12, **dadurch gekennzeichnet, dass** die Länge der Stange (12) zwischen ca. 65 mm und ca. 500 mm, vorzugsweise zwischen 70 mm und 75 mm, liegt.

14. Applikator nach einem der Ansprüche 1 bis 13, **dadurch gekennzeichnet, dass** der Innendurchmesser der Stange (12) zwischen ca. 1 mm und ca. 19 mm, vorzugsweise zwischen 1 mm und 2,8 mm, liegt.

15. Applikator nach einem der Ansprüche 1 bis 13, **dadurch gekennzeichnet, dass** der Außendurchmesser der Stange (12) zwischen ca. 2 mm und 20 mm liegt.

16. Applikator nach einem der Ansprüche 1 bis 15, **dadurch gekennzeichnet, dass** das erste Ende der Stange (12) mittels eines Klebstoffs verschlossen ist.

17. Applikator nach einem der Ansprüche 1 bis 16, **dadurch gekennzeichnet, dass** er des Weiteren einen Stopfen (30) aus flüssigem Material in der Nähe des offenen Endes der Stange aufweist, der die Verdunstung des Flüssigproduktes verhindern kann.

18. Verfahren zur Herstellung eines Applikators nach einem der Ansprüche 1 bis 17, **dadurch gekennzeichnet, dass** es die folgenden Schritte umfasst:

    - Übertragen einer Gruppe von hohlen Stangen zum Einlass einer Verschlussstation (32);
    - Verschließen eines der Enden der Stangen mittels eines Klebstoffes;
    - Füllen der Stangen mittels eines Flüssigproduktes durch ihr dem verschlossenen Ende gegenüberliegendes Ende;
    - Befestigen eines Endstücks (22) aus Fasermaterial an dem verschlossenen Ende jeder Stange und gleichzeitig Bilden eines Bereichs (28) mit verengtem Querschnitt an dem Endstück aus Fasermaterial,

    und dass es des Weiteren einen Schritt der Herstellung eines zerbrechbaren Bereichs (26) mit einer geringeren Festigkeit in der Wand jeder Stange in dem verschlossenen Endbereich umfasst, wobei der Bereich mit verengtem Querschnitt um den zerbrechbaren Bereich herum gebildet ist.

19. Verfahren nach Anspruch 18, **dadurch gekennzeichnet, dass** während des Schritts der Befestigung des Endstücks ein Endstück (18) zum Aufbringen von Produkt an dem offenen Ende jeder Stange befestigt wird.

**20.** Verfahren nach Anspruch 18 oder 19, **dadurch gekennzeichnet, dass** die Befestigung des Endstücks oder der Endstücke das Verkleben mindestens eines der Enden der Stangen und das Wickeln eines Faserstrangs auf das oder jedes verklebte Ende umfasst.

**21.** Verfahren nach einem der Ansprüche 18 bis 20, **dadurch gekennzeichnet, dass** es des Weiteren eine Verpakkungsphase umfasst, während der die fertigen Applikatoren (10) in Gruppen von mindestens einem Applikator in Verpackungen verpackt werden.

**22.** Verfahren nach einem der Ansprüche 18 bis 21, **dadurch gekennzeichnet, dass** es des Weiteren eine Phase der Sterilisation der fertigen Applikatoren umfasst.

**23.** Maschine zur Herstellung von Applikatoren nach einem der Ansprüche 1 bis 17, die eine Station (32) zum Verschließen von hohlen Stangen zum Verschluss eines der einander gegenüberliegenden Enden der Stangen, mindestens eine Station (34) zum Füllen der verschlossenen Stangen, eine Station (35) zum Bilden von Endstücken aus Fasermaterial zumindest an dem verschlossenen Ende der Stangen und eine Fördereinrichtung (40) zur Übertragung der Stangen von der Verschlussstation zur Befestigungsstation über die Füllstation umfasst, **dadurch gekennzeichnet, dass** die Station zum Bilden von Endstücken (35) eine Vorrichtung zum Bilden eines zerbrechbaren Bereichs (26) mit einer geringeren Festigkeit an dem verschlossenen Ende der Stangen und Mittel zum Bilden eines Bereichs mit einem verengten Querschnitt an dem Endstück um den zerbrechbaren Bereich herum umfasst.

FIG_1

FIG_2

FIG_3

FIG_4

FIG_5

FIG_6

FIG_7

FIG_8

**EP 1 594 563 B1**

**Documents brevets cités dans la description**

- US 5702035 A